(19) Europäisches Patentamt
European Patent Office
Office européen des brevets

(11) Publication number: **0 408 371 B1**

(12) # EUROPEAN PATENT SPECIFICATION

(45) Date of publication of patent specification: **28.09.94**

(51) Int. Cl.5: **C07K 7/06**, A61K 37/02

(21) Application number: **90307657.8**

(22) Date of filing: **12.07.90**

(54) **Hemoregulatory peptides.**

(30) Priority: **14.07.89 US 380578**

(43) Date of publication of application:
**16.01.91 Bulletin 91/03**

(45) Publication of the grant of the patent:
**28.09.94 Bulletin 94/39**

(84) Designated Contracting States:
**AT BE CH DE DK ES FR GB GR IT LI LU NL SE**

(56) References cited:
**EP-A- 0 000 053**
**EP-A- 0 267 741**

(73) Proprietor: **SMITHKLINE BEECHAM CORPORATION**
**P.O. Box 7929**
**1 Franklin Plaza**
**Philadelphia Pennsylvania 19101 (US)**

(72) Inventor: **Bhatnagar, Pradip Kumar**
**300 South Balderston Drive**
**Exton, PA 19341 (US)**
Inventor: **Huffman, William Francis**
**40 Crest Avenue**
**Malvern, PA 19335 (US)**
Inventor: **Talmadge, James Edward**
**22 James Thomas Road**
**Malvern, PA 19355 (US)**

(74) Representative: **Walker, Ralph Francis et al**
**SmithKline Beecham**
**Corporate Patents**
**Great Burgh**
**Yew Tree Bottom Road**
**Epsom Surrey KT18 5XO (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 408 371 B1

## Description

### Field of the Invention

The present invention relates to novel peptides which have hemoregulatory activities and can be used to stimulate haematopoiesis and for the treatment of viral, fungal and bacterial infectious diseases.

### Background of the Invention

A variety of regulatory messengers and modifiers such as colony stimulating factors, interferons, and different types of peptides are responsible for the regulation of myelopoiesis. Metcalf, Cell, 43:5 (1985); Baserga R., Foa P., Metcalf D, Polli EE (eds), Biological Regulation of Cell Proliferation (1986); Nicola et al., J. Cell Physiol. 128:501 (1986), Zoumbos et al., Proyr. Hemat. 1:341 and 14:201 (1986); Werner et al., Experientia 42:521 (1986). Twenty years ago, Rytomaa and Kivieniemi Cell Tissue Kinet 1:329-340 (1968); Rytomaa et al., Control of Cellular Growth in Adult Organisms pp 106-138 (1967) reported that extracts of mature granulocytes (granulocytic chalone) could specifically inhibit rat myelopoietic cell proliferation in cover slip cultures. Later, they demonstrated that the factor, which had a molecular weight less than 3,000 daltons, was able to induce the regression of a transplantable rat granulocytic leukemia, as well as retard the growth of leukemia cells in humans. Paukovits and others extracted a similar factor from rat bone marrow cells and showed that it inhibited the tritiated thymidine uptake of bone marrow cells, Paukovits, W.R., Cell Tissue Kinet 4:539-547 (1971); Naturforsch 37:1297 (1982). In 1979, Boll et al., Acta Haematologica 6:130 (1979) demonstrated the inhibitory effects of rat granulocytic extracts on human bone marrow cells in culture and a number of other researchers demonstrated that this crude granulocytic extract inhibited the development of g-CFUC and/or gm-CFUC in vitro from rodent bone marrow cells.

This biological agent was termed a granulocyte chalone which, according to this theoretical concept, should be an endogenous inhibitor of cell proliferation acting in the same tissue as it was secreted. The material obtained from crude extracts was found to be non-species specific but highly tissue specific. Furthermore, it was found to be nontoxic and to have reversible activities.

In 1982, a synthetic hemoregulatory pentapeptide having the following structure:

pGlu-Glu-Asp-Cys-Lys

was reported to have a selective inhibitory effect on myelopoietic cells both in vitro and in vivo, where the main effect seems to be on myelopoietic stem cells (CFU-gm), Paukovits et al., Z. Naturforsch 37:1297 (1982) and U.S. Patent No. 4,499,081. This peptide is believed to be an analogue of a naturally occurring granulopoiesis inhibition factor which has been found in minute quantities in bone marrow extracts. The peptide, by inhibiting haematopoiesis, and, in particular, granulopoiesis tends to prevent quiescent cells from entering into cell division and so becoming susceptible to attack by cytotoxic anti cancer drugs. In addition to providing a protective function in therapy using cytotoxic drugs, the peptides may also be used to arrest proliferation of cancer cells related to the myelopoietic system, i.e. myeloleukaemia.

In 1987, Laerum et al., reported that the oxidation product of this peptide was a dimer (HP-5) formed by disulfide bridges. This dimer has the opposite effect as the monomer as it strongly stimulates colony formation of both human and murine CFU-gm in vitro and up regulates murine myelopoietic cells in vivo. It is claimed in European Application EP-A-0267741.

The dimer is reported as being useful in stimulating myelopoiesis in patients suffering from reduced myelopoietic activity, including bone marrow damage, agranulocytosis and aplastic anemia including patients having depressed bone marrow function due to immunosuppressive treatment to suppress tissue reactions i.e. in bone marrow transplant surgery. The compounds may also be used to promote more rapid regeneration of bone marrow after cytostatic chemotherapy and radiation therapy for neoplastic and viral diseases. Then may be of particular value where patients have serious infections due to a lack of immune response following bone marrow failure.

The presence of the disulfide bond in the HP-5 dimer suggests that the monomer is a possible metabolite in vivo. Since the monomer inhibits haematopoiesis, the stability of the HP-5 dimer is critical when used in vivo to stimulate myelopoiesis. The identification of a stable dimer would eliminate this potential problem.

### Summary of the Invention

This invention comprises peptides, hereinafter represented as formula (I), which have hemoregulatory activities and can be used to stimulate haematopoiesis and treat bacterial, viral and fungal diseases. These

peptides are useful in the restoration of leukocytes in patients with lowered cell counts resulting from a variety of clinical situations, such as surgical induced myelosuppression, AIDS, congenital myelodysplacis, bone marrow and organ transplants; in the protection of patients with leukopenia from infection; in the treatment of severely burned patients and in the amelioration of the myelosuppression observed with some cell-cycle specific antiviral agents. The peptides are also useful in the treatment of viral, fungal and bacterial infectious diseases, particularly Candida and Herpes in both immunosuppressed and "normal" subjects.

These compounds may also be used in combination with the monomers of U.S. Patent No. 4,499,081 to provide alternating peaks of high and low activity in the bone marrow cells, thus augmenting the natural circadian rhythm of haematopoiesis. In this way, cytostatic therapy can be given at periods of low bone marrow activity, thus reducing the risk of bone marrow damage, while regeneration will be promoted by the succeeding peak of activity.

This invention is also a pharmaceutical composition, which comprises a compound of formula (I) and a pharmaceutically acceptable carrier.

The compounds of this invention may be used in a method for stimulating the myelopoetic system of an animal, including humans, which comprises administering to an animal in need thereof, an effective amount of a compound of formula (I).

The compounds of this invention may be used in a method for treating viral, fungal and bacterial infections in immunosuppressed and normal animals, including humans, which comprises administering to an animal in need thereof, an effective amount of a compound of formula (I).

## Detailed Description of the invention

The peptides of this invention are illustrated by the formula (I):

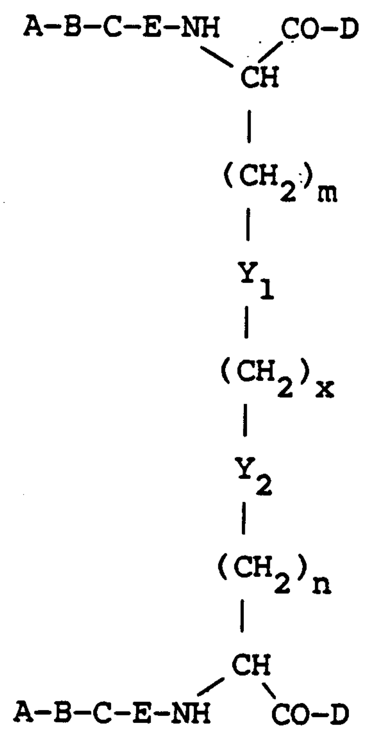

I

$Y_1$ and $Y_2$ are independently $CH_2$ or S;
x is 0, 1, 2, 3 or 4;
m is 0, 1 or 2;
n is 0, 1, or 2;
A is pyroglutamic acid, proline, glutamine, tyrosine or glutamic acid;
B is glutamic acid, tyrosine or aspartic acid;
C is glutamic acid, tyrosine or aspartic acid;
D is lysine, arginine, tyrosine, N-methyl arginine, diaminohexynoic acid or the carboxyamide, or

hydroxy methyl derivative thereof;

E is glutamic acid, aspartic acid, tyrosine or a peptide bond;

provided that:

when $Y_1$ and $Y_2$ are S, x is 2, 3 or 4 and m and n are 1; or

when $Y_1$ and $Y_2$ are $CH_2$, x is 0, 1 or 2 and m and n are 0; or

when $Y_1$ is S and $Y_2$ is $CH_2$, x is 0 and n is 1; or

when $Y_2$ is S and $Y_1$ is $CH_2$, x is 0 and m is 1;

or a pharmaceutically acceptable salt thereof.

Also included in this invention are pharmaceutically acceptable salt complexes of the compounds of this invention. It should be noted in formula (I) that A comprises the terminal amino group of the amino acid residue corresponding to pyroglutamic acid proline, glutamine, tyrosine or glutamic acid. Similarly, D comprises the terminal carboxyl group of amino acid residue corresponding to lysine, arginine, tyrosine, N-methyl arginine, diamino hexynoic acid or the carboxamide or hydroxy methyl derivative thereof.

The abbreviations and symbols commonly used in the art are used herein to describe the peptides:

| | |
|---|---|
| pGlu = | pyroglutamic acid |
| Pro = | proline |
| Gln = | glutamine |
| Glu = | glutamic acid |
| Asp = | aspartic acid |
| Lys = | lysine |
| Arg = | arginine |
| Cys = | cysteine |
| Tyr = | tyrosine |
| Sub = | diaminosuberic acid |
| Hna = | diaminohexynoic acid |
| Pim = | diaminopimelic acid |
| Adp = | diamino adipic acid |

In accordance with conventional representation, the amino terminus is on the left and the carboxy terminus is on the right. All chiral amino acids may be in the D or L absolute configuration.

The amino terminus may be protected by acylation. Examples of such protecting groups are, t-butoxycarbonyl (t-Boc), $CH_3CO$ and Ar-CO (Ar = benzyl).

The C-terminus may be carboxy as in the case of the natural amino acid or the carboxyamide

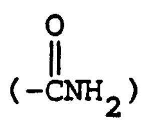

or hydroxy methyl ($-CH_2-OH$).

Preferred compounds are those in which Y and $Y_2$ are $CH_2$ and x is 1 or 2 or wherein A is pGlu, B is Glu, C is Asp, D is Lys and E is a peptide bond and the chiral amino acids are in the L absolute configuration.

Especially preferred compounds are:

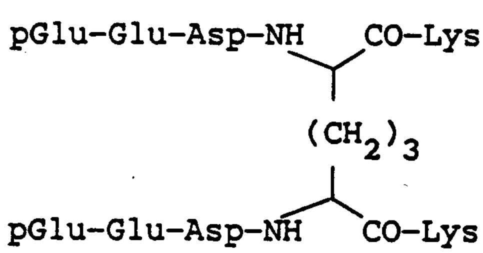

and

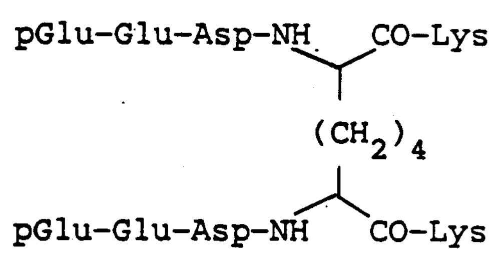

The compounds of this invention differ from those of the prior art in that the dimer is linked by a bridge that contains a carbon-carbon bond rather than a disulfide bridge. The carbon-carbon bond is not readily cleaved in vivo and, therefore, is more stable in vivo than the compounds of European Application EP-A-0267741.

The peptides of the invention are prepared by the solid phase technique of Merrifield, J. Am. Chem. Soc., 85, 2149 (1964), or solution methods known to the art may be successfully employed. The methods of peptide synthesis generally set forth in J. M. Stewart and J. D. Young, "Solid Phase Peptide Synthesis", Pierce Chemical Company, Rockford, Il (1984) or M. Bodansky, Y. A. Klauser and M. A. Ondetti, "Peptide Synthesis", John Wiley & Sons, Inc., New York, N.Y. (1976) may be used to produce the peptides of this invention and are incorporated herein by reference.

Each amino acid or peptide is suitably protected as known in the peptide art. For example, the fluorenylmethoxy carbonyl radical (Fmoc) or t-butoxycarbonyl (t-Boc) group are preferred for protection of the amino group, especially at the $\alpha$-position. A suitably substituted carbobenzyloxy group may be used for the $\epsilon$-amino group of lysine and benzyl group for the $\beta$ and $\gamma$ carboxy groups Asp and Glu respectively. Suitable substitution of the carbobenzyloxy lu protecting group is ortho and/or para substitution with chloro, bromo, nitro or methyl, and is used to modify the reactivity of the protective group. Except for the t-Boc group, the protective groups are, most conveniently, those which are not removed by mild acid treatment. These protective groups are removed by such methods as catalytic hydrogenation, sodium in liquid ammonia or HF treatment as known in the art.

If solid phase methods are used, the peptide is built up sequentially starting from the carboxy terminus and working toward the amino terminus of the peptide. Solid phase synthesis is begun by covalently attaching the C terminus of a protected amino acid to a suitable resin, such as a benzhydrylamine resin (BHA), methylbenzhydrylamine resin (MBHA) or chloromethyl resin (CMR), as is generally set forth in U.S. Patent No. 4,244,946 or phenylacetamidomethyl resin (PAM). A BHA or MBHA support resin is used if the carboxy terminus of the product peptide is to be a carboxamide. A CMR or Pam resin is generally used if the carboxy terminus of the product peptide is to be a carboxy group, although this may also be used to produce a carboxamide or ester.

The protective group on the $\alpha$-amino group is removed by mild acid treatment (i.e. trifluoroacetic acid). For compounds in which $Y_1$ and/or $Y_2$ are $CH_2$ instead of S, a di-Boc (diaminodicarboxcylic acid) is coupled to two of the amino acids on the resin using a suitable coupling agent. Any free carboxyl groups are amidated with suitable protected derivative of D. Suitable deprotection, neutralization and coupling cycles known in the art are used to sequentially add amino acids without isolation of the intermediate, until the desired peptide has been formed. The completed peptide may then be deblocked and/or split from the carrying resin in any order.

Treatment of a resin supported peptide with HF or HBr/acetic acid splits the peptide from the resin and produces the carboxy terminal amino acid as a carboxylic acid.

If an ester is desired, the CMR or Pam resin may be treated with an appropriate alcohol, such as methyl, ethyl, propyl, butyl or benzyl alcohol, in the presence of triethyiamine to cleave the peptide from the resin and produce the ester directly.

Esters of the peptides of this invention may also be prepared by conventional methods from the carboxylic acid precursor. Typically, the carboxylic acid is treated with an alcohol in the presence of an acid catalyst. Alternatively, the carboxylic acid may be converted to an activated acyl intermediate, such as an acid halide, and treated with an alcohol, preferably in the presence of a base.

The preferred method for cleaving a peptide from the support resin is to treat the resin supported peptide with anhydrous HF in the presence of a suitable cation scavenger, such as anisole or dimethoxybenzene. This method simultaneously removes all protecting groups, except a thioalkyl group protecting sulfur, and splits the peptide from the resin. Peptides hydrolyzed in this way from the CMR and Pam resins are carboxylic acids, those split from the BHA resin are obtained as carboxamides.

Modification of the terminal amino group of the peptide is accomplished by alkylation or acylation as is generally known in the art. These modifications may be carried out upon the amino acid prior to incorporation into the peptide, or upon the peptide after it has been synthesized and the terminal amino group liberated, but before the protecting groups have been removed.

Typically, acylation is carried out upon the free amino group using the acyl halide, anyhydride or activated ester, of the corresponding alkyl or aryl acid, in the presence of a tertiary amine. Mono-alkylation is carried out most conveniently by reductive alkylation of the amino group with an appropriate aliphatic aldehyde or ketone in the presence of a mild reducing agent, such as lithium or sodium cyanoborohydride. Dialkylation may be carried out by treating the amino group with an excess of an alkyl halide in the presence of a base.

Solution synthesis of peptides is accomplished using conventional methods used to form amide bonds. Typically, a protected t-Boc amino acid which has a free carboxyl group is coupled to a protected amino acid which has a free amino group using a suitable coupling agent, such as N, N′ dicyclohexyl carbodiimide

(DCC), optionally in the presence of catalysts such as 1-hydroxybenzotriazole (HOBT) or dimethylamino pyridine (DMAP). Other methods, such as the formation of activated esters, anhydrides or acid halides, of the free carboxyl of a protected t-Boc-amino acid, and subsequent reaction with the free amine of a protected amino acid, optionally in the presence of a base, are also suitable. For example, a protected Boc-amino acid or peptide is treated in an anhydrous solvent, such as methylene chloride or tetrahydrofuran (THF), in the presence of a base, such as N-methyl morpholine, DMAP (dimethylaminopyridine) or a trialkyl amine, with isobutyl chloroformate to form the "activated anydride", which is subsequently reacted with the free amine of another protected amino acid or peptide. The peptide formed by these methods may be deprotected selectively, using conventional techniques, at the amino or carboxy terminus and coupled to other peptides or amino acids using similar techniques. After the peptide has been completed, the protecting groups may be removed as hereinbefore described, such as by hydrogenation in the presence of a palladium or platinum catalyst, treatment with sodium in liquid ammonia, hydrofluoric acid or alkali.

If the final peptide, after it has been deprotected, contains a basic group, an acid addition salt may be prepared. Acid addition salts of the peptides are prepared in a standard manner in a suitable solvent from the parent compound and an excess of an acid, such as hydrochloric, hydrobromic, sulfuric, phosphoric, acetic, maleic, succinic or methanesulfonic. The acetate salt form is especially useful. If the final peptide contains an acidic group, cationic salts may be prepared. Typically the parent compound is treated with an excess of an alkaline reagent, such as a hydroxide, carbonate or alkoxide, containing the appropriate cation. Cations such as $Na^+$, $K^+$, $Ca^{++}$ and $NH_4^+$ are examples of cations present in pharmaceutically acceptable salts. $Na^+$ and $NH_4^+$ are especially preferred.

In general, in order to exert a stimulatory effect, the peptides of the invention may be administered to human patients by injection or orally in the dosed range 0.1-10 mg, for example 1-5 mg per 70 kg body weight per day, if administered by infusion or similar techniques, the dose may be in the range 30-300 mg per 70 kg body weight, for example about 100 mg over six days. In principle, it is desirable to produce a concentration of the peptide of about $10^{-13}$M to $10^{-5}$M in the extracellular fluid of the patient.

According to a still further feature of the present invention there are provided pharmaceutical compositions comprising as active ingredient one or more compound of formula (I) as hereinbefore defined or physiologically compatible salts thereof, in association with a pharmaceutical carrier or excipient. The compositions according to the invention may be presented, for example, in a form suitable for oral, nasal, parenteral or rectal administration.

As used herein, the term "pharmaceutical" includes veterinary applications of the invention.

These peptides may be encapsulated, tableted or prepared in an emulsion or syrup for oral administration. Pharmaceutically acceptable solid or liquid carriers may be added to enhance or stabilize the composition, or to facilitate preparation of the composition. Liquid carriers include syrup, peanut oil, olive oil, glycerin, saline and water. Solid carriers include starch, lactose, calcium sulfate dihydrate, terra alba, magnesium stearate or stearic acid, talc, pectin, acacia, agar or gelatin. The carrier may also include a sustained release material such as glyceryl monostearate or glyceryl distearate, alone or with a wax. The amount of solid carrier varies but, preferably will be between about 20 mg to about 1 g per dosage unit. The pharmaceutical preparations are made following the conventional techniques of pharmacy involving milling, mixing, granulating, and compressing, when necessary for tablet forms; or milling, mixing and filling for hard gelatin capsule forms. When a liquid carrier is used the preparation will be in the form of a syrup, elixir, emulsion or an aqueous or non-aqueous suspension. Such a liquid formulation may be administered directly p.o. or filled into a soft gelatin capsule. Organ specific carrier systems may also be used.

Alternately pharmaceutical compositions of the peptides of this invention, or derivatives thereof, may be formulated as solutions or lyophilized powders for parenteral administration. Powders may be reconstituted by addition of a suitable diluent or other pharmaceutically acceptable carrier prior to use. The liquid formulation is generally a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

For rectal administration, a pulverized powder of the peptides of this invention may be combined with excipients such as cocoa butter, glycerin, gelatin or polyethylene glycols and molded into a suppository. The pulverized powders may also be compounded with an oily preparation, gel, cream or emulsion, buffered or unbuffered, and administered through a transdermal patch.

Nasal sprays may be formulated similarly in aqueous solution and packed into spray containers either with an aerosol propellant or provided with means for manual compression. Capsules containing one or

several active ingredients may be produced, for example by mixing the active ingredients with inert carriers, such as lactose or sorbitol, and filling the mixture into gelatin capsules.

Dosage units containing the compounds of this invention preferably contain 0.1-10 mg, for example 1-5 mg of the peptide of formula (I) or salt thereof.

According to a still further feature of the present invention there is provided a method of stimulation of myelopoiesis which comprises administering an effective amount of a pharmaceutical composition as hereinbefore defined to a subject.

Another feature of the present invention provides a method for treating viral, fungal and bacterial infections in immunosuppressed and normal animals which comprises administering to an animal in need thereof, an effective amount of a pharmaceutical composition as hereinbefore defined.

The biological activity of the compounds of formula I are demonstrated by the following test.

Induction of Colony Stimulating Activity by Stromal Cells

The human bone marrow stromal cell line, C6, is grown to confluency in plastic tissue culture dishes in RPMI-1640 medium and 5% FBS. On the day prior to the experiment this medium is changed to DMEM without added serum. To these cultures, the compounds are added for one hour, then washed from the cultures. The medium is replaced with fresh DMEM and the cells are incubated for 24 hours at 37 ° C, 5% $CO_2$. After 24 hours the C6 cell culture supernatant is collected, sterile filtered, and frozen until it can be assayed for the presence of hematopoietic colony stimulating activity (CSA) as set forth below.

Soft Agar Assay

Bone marrow cells are obtained from Lewis rats. They are adjusted to $10^6$ cells/ml in DMEM without serum. A single later agar system utilizing the following is used: DMEM enriched with nutrients ($NaHCO_3$, pyruvate, amino acids, vitamins, and HEPES buffer); 0.3% Bacto agar, and 20% Lewis rat serum. To this are added dilutions of C6 cell line supernatant (10-2.5%) from above along with rat bone marrow cells (final concentration = $10^5$ cells/ml). The agar plates are incubated at 37 ° C, 5% $CO_2$ for 7-8 days. Colonies of proliferating bone marrow cells (CFU-C) are counted utilizing a microscope. The number of agar colonies counted is proportional to the amount of CSA present within the C6 bone marrow stromal cell line supernatant.

TABLE 1

| Hematopoietic Colony Stimulating Activity Using 5% Supernatant | |
|---|---|
| Dose ng/ml | Percent of Control $(pGlu\text{-}Glu\text{-}Asp)_2\text{-}Sub\text{-}(Lys)_2$ (Example 2) |
| 1000 | 192 |
| 100 | 241 |
| 10 | 207 |
| 1 | 188 |
| 0.1 | 154 |
| 0.01 | 97 |
| 0.001 | - |

Herpes Simplex Mouse Model

Seven days prior to infection, Balb/c mice are injected intraperitoneally once a day with a 0.2 ml volume at doses of 10 and 1 ng/kg of compound. Control mice receive injections of 0.2 ml of a mixture of the dilution buffer, DPBS and 0.5% heat inactivated normal mouse serum.

The mice are infected with a Herpes Simplex virus (strain MS) by injecting 5.0 x $10^5$/pfu suspended in 0.05 mls of PBS in each rear foot pad. The mice continue to get compound or control injections until moribund (unable to get food or water). Usually paralysis of the hind leg occurs approximately eight days after infection. The paralysis progresses until encephalitis occurs.

Alternatively, the virus is inoculated by means of a vaginal route. A cotton plug containing 5.0 x $10^5$/pfu of the MS-NAP strain is inserted into the vagina of the mouse.

A Wilcoxin test is used to determine if a significant increase in survival is found in the treated verses control group.

Candida challenge

Candida albicans strain B311a is used. This strain has been mouse passed then frozen at -70 °C. B311a is virulent to immunosuppressed mice in the range of 5.0 to 8.0 x $10^4$ cfu/mouse and for normal mice in the range of 1.0 to 2.0 x $10^5$ cfu/mouse. A sample from the frozen stock of Candida was grown on Sabouraud dextrose slants and then transferred to 50 ml. shake cultures of Sabouroud broth for 18 hours. The cells were washed three times, then counted by hemocytometer, and viability was confirmed by methylene dye exclusion. Vialbility counts were performed on the inoculum to confirm the counts.

All mice (Balb/c) infected with Candida were infected i.v. with cells suspended in 0.2 mls. of saline. Some mice are sublethally myelodepressed with 300 rads of irradiation. Beginning 2 hours following irradiation, the animals are injected with compound CSF as a positive control, or excipient, daily. Seven days after irradiation and treatment begins, the mice are challenged with Candida albicans by intravenous administration. Note that this represents approximately a $LD_{75}$ for normal mice. In other studies the mice are not immunosuppressed. In these studies the mice are treated starting seven days post infection in the same manner as the irradiated mice. In both models the mice are followed until moribund and the change is survival compared using the Wilcoxin test.

The examples which follow serve to illustrate this inventions The Examples are intended to in no way limit the scope of this invention, but are provided to show how to make and use the compounds of this invention.

In the Examples, all temperatures are in degrees Centigrade. Amino acid analysis were performed upon a Dionex Autoion 100. Analysis for peptide content is based upon Amino Acid Analysis. FAB mass spectra were performed upon a VG ZAB mass spectrometer using fast atom bombardment. The abbreviations used are as follows:

| | |
|---|---|
| Arg = | arginine |
| Asp = | aspartic acid |
| t-BOC = | tert. butyloxy carbonyl |
| Bz = | benzyl |
| Cl-Z = | p-chloro carbobenzyloxy carbonyl (Z = carbobenzyloxy carbonyl) |
| DCC = | dicylohexyl carbodiimide |
| DIEA = | diisopropylethyl amine |
| EDC = | (N ethyl-N'-(3-dimethylaminopropyl) carbodiimide |
| Glu = | glutamic acid |
| p-Glu = | pyroglutamic acid |
| Tyr = | tyrosine |
| Hna = | diaminohexynoic acid |
| HOBT = | hydroxybenzotriazole |
| Lys = | lysine |
| NMP = | N methyl 2-pyrrolidinone |
| Pro = | proline |
| Gln = | glutamine |
| Cys = | cysteine |
| Pim = | |

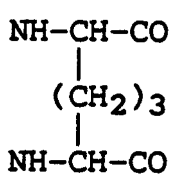 (diaminopimelic acid)

Sub =

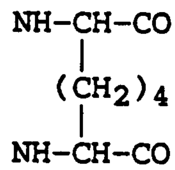 (diaminosuberic acid)

Adp =

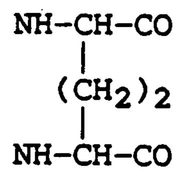

| | |
|---|---|
| N-meArg = | N-methyl arginine |
| Prc = | bis BOC-S,S'-1,3-propanediylcysteine |
| Etc = | bis BOC-S,S'-1,2-ethanediylcysteine |
| Buc = | bis BOC-S,S'-1,4-butanediylcysteine |

EXAMPLE 1

Preparation of: (p-Glu-Glu-Asp)$_2$-Pim-(Lys)$_2$

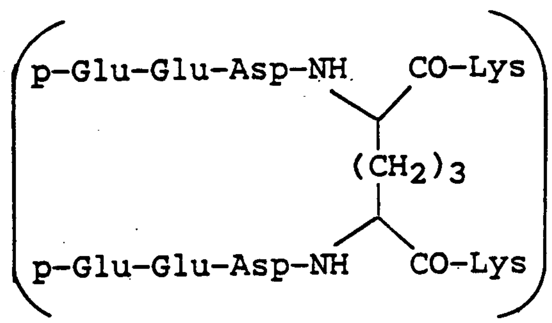

A half gram of t-BOC-Lys(Cl-Z)-O $CH_2$-Pam Resin (0.63 m mol/gm) was loaded in the reaction vessel of a Beckman 990 B synthesizer. In the deprotection step, the t-Boc group was removed using 40% trifluoroacetic acid (TFA) in methylene chloride ($CH_2Cl_2$). The trifluoroacetate salt was neutralized by 10% DIEA/$CH_2Cl_2$. Two mg (780 mgs) of Di-BOC-2,6-diaminopimelic acid was coupled using 2 mM of DCC and HOBT. The coupling was done in the mixture of 15 ml of $CH_2Cl_2$ and 10 ml of DMF at room temperature for two hours. Kaiser's test was used to monitor the coupling. Any remaining free carboxyl groups were amidated twice by using 3 mM (1.65 gms) of H-Lys(Z)-OBz.HCl and 3 mM of DCC and 3mM of HOBT in 25 ml of $CH_2Cl_2$/DMF (15/10).

After two hours of coupling, the resin was washed twice with 15 ml of $CH_2Cl_2$, twice with 15 ml of DMF, twice with 15 ml of MeOH/$CH_2Cl_2$ (1:1), and finally twice with 15 ml of $CH_2Cl_2$. After the deprotection of t-Boc using 40% TFA/$CH_2Cl_2$ and the neutralization using 10% DIEA/$CH_2Cl_2$, 2 mM (0.646 gm) of Boc-Asp-(Bzl) and 2 mM of DCC and 2 mM of HOBT were added and coupled for 2 hours in 25 ml of $CH_2Cl_2$/DMF (15/10). The resin was then subjected to a washing step as described earlier. The deprotection step and the neutralization step were repeated before 2 mM (0.674 gm) of Boc-Glu (Bzl), 2 mM DCC and 2 mM of HOBT were coupled in 25 ml of $CH_2Cl_2$/DMF (15/10). After washing, deprotection and neutralization steps, 2mM (0.258 gm) of pGlu, 2mM of DCC and 2mM of HOBT were coupled in 25 ml of $CH_2Cl_2$/DMF (15/10) for 2 hours before the resin was subjected to a washing step. Completion of the coupling was monitored by Kaiser's test and only single coupling was needed at each step. After the completion of the synthesis the

resin was dried and weighed. Yield: 1.2 g.

The peptide resin (1.2 gm) was charged in a cleavage apparatus and cleaved using 10 ml of hydrofluoric acid (HF) and 1 ml anisole at -15°C for two hours. After removal of HF under vacuum the mixture of resin and peptide was extensively washed with ether and the peptide was extracted in glacial acetic acid (30 ml). Most of the acid was removed from the extracts on a rotavap and the residue was diluted in water and lyophilized. The acetic acid extract had 810 mgs of crude peptide.

The crude peptide (80 mgs), obtained from acetic acid extraction, was further purified using a preparative C-18 column. It was passed through a pre-equilibrated (in 0.1% TFA/$H_2O$) column. The peptide was eluted using a linear gradient of 80% acetonitrile, 20% $H_2O$ and 0.1% TFA.

Three isomers co-eluted (8.52 min). These were separated on a C-18 column using a gradient of 30% (0.1% TFA in $CH_3CN$), 70% (0.1% TFA in $H_20$) to 80% (0.1% TFA in $CH_3CN$), 20% (0.1% TFA in $H_2O$) over 30 minutes at a flow rate of 1.5 ml/min. The following fractions were eluted:

fraction 1: 18.69 min
fraction 2: 19.68 min
fraction 3: 22.95 min

Amino acid analysis gave the following results:

| Amino Acid Analysis | Observed |
|---|---|
| Glu | 1.99 |
| Asp | 1.0 |
| Lys | 1.05 |
| Bis amino pimelic acid | N.D. |
| mass spec = 1157.5 $(M+H)^+$ | |

EXAMPLE 2

Preparation of: $(pGlu\text{-}Glu\text{-}Asp)_2\text{-}Sub\text{-}(Lys)_2$

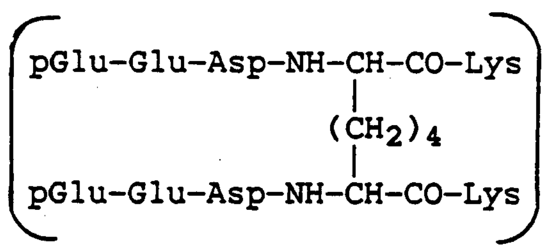

A. Synthesis of BOC-SUB-Lys-(ε-Z)COOBz.:

Bis-BOC (1,1) diaminosuberic acid (Sub) was synthesized using R. Nutt's method (J. Org. Chem. 45, 3078, 1980).

Two mM of Boc-Sub (808 mg), 4mM of Lys-(ε-Z)-COOBz.HCl (1.56 g) and 4mM of HOBT (0.613 g) were dissolved in 10 ml of methylene chloride ($CH_2Cl_2$) and the solution was chilled to -15°C using an ice/acetone bath. Four mM (0.692 ml) of diisopropyl ethyl amine (DIEA) were added followed by the addition of 0.772 g (4 mM) water soluble carbodiimide (EDC). After stirring for one hour the mixture was allowed to warm to room temperature. After three hours the methylene chloride was evaporated and the residue was dissolved in 200 ml of ethyl acetate. The solution was washed first with 1N HCL, then 1N NaOH, saturated NaCl solution and water. The washes were repeated three times and each wash was about 100 ml. The organic later was dried over $MgSO_4$ and evaporated. 1.86 g of BOC-Sub-(ε-Z)Lys-COOBz (79% yield) was obtained and used further without any purification.

B. Synthesis of BOC Asp-(β-OBz)Sub Lys-(ε-Z)-COOBz).:

BOC-Sub-Lys(ε-Z)-COOBz (1.8 g) was dissolved in 4N HCl-dioxane for a half hour and then evaporated to dryness. The residue was washed with ether and dried overnight. The hydrochloride salt was dissolved in 30 ml of $CH_2Cl_2$ and BOCAsp-(β-OBz) (1.292 g) was added. The solution was chilled to -15° C and 0.613 g HOBT, 0.554 ml DIEA and 0.772 g of EDC were added. After stirring for two hours the mixture was allowed to warm up to room temperature. After 18 hours (overnight) the reaction mixture was worked up. $CH_2Cl_2$ was evaporated and the residue was dissolved in 200 ml of ethyl acetate. The solution was washed with 1N HCl, 1N NaOH, saturated NaCl solution and water (washes were repeated three times and each wash was about 100 ml). The organic layer was dried over $MgSO_4$ and evaporated. BOC-Asp-(β-OBz)-Sub-Lys-(ε-Z)-COOBz 1.9 g (yield 73%). This peptide was used without any further purification.

C. Synthesis of BOC-Glu-(γ-OBz) Asp-(β-OBz)Sub-Lys-(ε-Z)COOBz.:

BOC-(β-OBz) Asp-Sub-(ε-Z) Lys-COOBz. 1.8 g was dissolved in 15 ml of 4N HCl dioxane. After fifteen minutes the solvent was removed and the residue was washed with ether and dried. The hydrochloride salt was dissolved in 15 ml of N-methyl pyrrolidone (NMP). The solution is chilled to -15° C and 4 mM (1.338) of BOC-Glu(γ-OBz), 0.204 ml DIEA, 0.772 g EDC and 0.612 g of HOBT were added. The mixture was stirred over night while gradually warming up to room temperature. The reaction mixture was added to a flask containing one liter of chilled 10% $Na_2CO_3$ in saturated NaCl solution. The precipitates were filtered, washed with water, and dried under vacuum. BOC-(γ-OBz)Glu-(β-OBz)Asp-Sub-(ε-Z)Lys-COOBz (1.3 g) was obtained and used without any further purification. Yield: 68%.

D. Synthesis of pGlu-(γ-OBz) Glu-(β-OBz) Asp-Sub-(ε-Z) Lys-COOBz.:

BOC-(γ-OBz) Glu-(β-OBz)Asp-Sub-(ε-Z)Lys-COOBz 1.2 g was dissolved in 15 ml of 4N HCl dioxane. After fifteen. minutes, the solvent was removed and the residue was washed with ether and dried. The hydrochloride salt was dissolved in 15 ml of NMP. The solution is chilled to -15° C and 4 mM (0.516 g) pyro-Glu(p-Glu), 0.106 ml DIEA, 0.772 g EDC and 0.612 g of HOBT were added. The mixture was stirred overnight while gradually warming up to room temperature. The reaction mixture was added to a flask containing one liter of chilled 10% $Na_2CO_3$ in saturated NaCl solution. The precipitates were filtered washed with water and dried under vacuum. pGlu-(γ-OBz) Glu-(β-OBz) Asp-Sub-(ε-Z) Lys-COOBz (0.830G) was obtained and used without any further purification. Yield: 69%.

E. Synthesis of pGlu-Glu-Asp-Sub-Lys-COOH:

pGlu-(γ-OBz) Glu-(β-OBz) Asp-Sub-(ε-Z) Lys-COOBz (0.200 g) was deprotected using 5 ml HF/1.5 ml anisole at 0° C. HF is removed and the peptide is partitioned between ether and 0.1 N acetic acid. The aqueous later is washed and lyophilized. pGlu-Glu-Asp-Sub-Lys-COOH 0.089 g is obtained. Twenty mgs of this peptide are purified on C18 prep Vyadec column using isocratic condition (10% acetonitrile, 90% water and 0.1 % trifluoroacetic acid, flow rate 5.6 ml/minute).
FAB Mass: M+H = 1171.4.Amino Acid Analysis: Asp (1.0), Glu (2.19), Lys (1.01), Sub N.D. HPLC: Retention time on C18 Vyadec 0.23x25 mm analytical column 7.01 minute [flow rate 1.5 ml gradient 0% to 80% B A = 0.1% TFA in water and B = 0.1% TFA in acetonitrile).

EXAMPLE 3

Preparation of $(pGlu\text{-}Glu\text{-}Asp)_2$-Lan-$(Lys)_2$ [Lan = Lanthionine($SCH_2CH(NH_2)COOH$)]

A half gram of t-BOC-Lys(Cl-Z)-$CH_2$ PAM (0.63 m. m/g) is charged in the reaction vessel of a Beckman 990 synthesizer. The t-BOC group is removed using 40% TFA in methylene chloride. The trifluoroacetic acid salt is neutralized by 10% DIEA/$CH_2Cl_2$. Two mM of Bis BOC lanthionine is coupled using 4 mM of DCC and HOBT in 15 ml of $CH_2Cl_2$ and 10 ml of DMF at room temperature. The Kaiser test is used to monitor the coupling. Any free remaining carboxyl groups are amidated using 3mM of H-Lys-O-Bz. HCl; and 3 mM of DCC and HOBT in 25 ml of $CH_2Cl_2$/DMF (15/10). After the coupling resin is extensively washed with $CH_2Cl_2$, 30% MeOH-$CH_2Cl_2$, and $CH_2Cl_2$ (25 ml x 3), the cycles of deprotection, neutralization and coupling are repeated with the remaining amino acids in the target peptide (Asp, Glu, pGlu). Four mM of each amino acid, DCC and HOBT are used for each coupling. Each coupling is monitored using Kaiser test.

After completion of the synthesis, the resin is dried and weighed.

The peptide resin is charged in cleavage apparatus and cleaved using 10 ml of hydrofluoric acid (HF) and one ml of anisole at -15 ° C for two hours. After removal of the HF, the resin is extensively washed with ether and the peptide is extracted with glacial acetic acid (30 ml). Most of the acetic acid is removed on a rotavap and the residue is diluted in water and lyophilized. After purification by HPLC, the peptide is obtained.

EXAMPLE 4

Preparation of $(Pro\text{-}Asp\text{-}Asp)_2\text{-}Sub\text{-}(Lys)_2$

A half gram of t-BOC-Lys(Cl-Z)-$CH_2$ Pam (0.63 m. M/g) is charged in the reaction vessel of a Beckman 990 synthesizer. The t-BOC group is removed using 40% TFA in methylene chloride. The trifluoroacetic acid salt is neutralized by 10% DIEA/$CH_2Cl_2$. Two mM of Bis BOC diamino suberic acid is coupled using 4 mM of DCC and HOBT in 15 ml of $CH_2Cl_2$ and 10 ml of DMF at room temperature. The Kaiser test is used to monitor the coupling. Any free remaining carboxyl groups are amidated using 3 mM of H-Lys-O-Bz. HCl; and 3 mM of DCC and HOBT in 25 ml $CH_2Cl_2$/DMF (15/10). After coupling, the resin is extensively washed with $CH_2Cl_2$, 30% MeOH-$CH_2Cl_2$, and $CH_2Cl_2$, and $CH_2Cl_2$ (25 ml x 3). The cycles of deprotection, neutralization and coupling are repeated with the remaining amino acids in the target peptide (Asp, Asp, and Pro). Four mM of amino acids DCC and HOBT are used for each coupling. Each coupling is monitored using the Kaiser test. After completion of the synthesis, the resin was dried and weighed.

The peptide resin is charged in a cleavage apparatus and cleaved using 10 ml of hydrofluoric acid (HF) and one ml of anisole at -15 ° C for two hours. After removal of the HF, the resin is extensively washed with ether and the peptide is extracted with glacial acetic acid (30 ml). Most of the acetic acid is removed on a rotavap and the residue is diluted in water and lyophilized. After purification by HPLC, the peptide is obtained.

EXAMPLE 5

Preparation of $(pGlu\text{-}Asp\text{-}Asp)_2\text{-}Pim\text{-}(Lys)_2$

A half gram of BOC-Lys(Cl-Z)-$CH_2$ PAM (0.63 m. M/g) is charged in the reaction vessel of a Beckman 990 synthesizer. The t-BOC group is removed using 40% TFA in methylene chloride. The trifluroacetic acid salt is neutralized by 10% DIEA/$CH_2Cl_2$. Two mM of Bis BOC pimelic acid is coupled using 4mM of DCC and HOBT in 15 ml of $CH_2Cl_2$ and 10 ml of DMF at room temperature. The Kaiser test is used to monitor the coupling. Any free remaining carboxyl groups are amidated using 3 mM of H-Lys-O-Bz. HCl; and 3 mM of DCC and HOBT in 25 ml of $CH_2Cl_2$/DMF (15/10). After coupling, the resin is extensively washed with $CH_2Cl_2$, 30% meOH-$CH_2Cl_2$, and $CH_2Cl_2$ (25 ml x 3). The cycles of deprotection, neutralization and coupling are repeated with the remaining amino acids in the target peptide (Asp, Asp, and p-Glu). Four mM of amino acid, DCC and HOBT are used for each coupling. Each coupling is monitored using the Kaiser test. After completion of the synthesis, the resin is dried and weighed.

The peptide resin is charged in a cleavage apparatus and cleaved using 10 ml of hydrofluoric acid (HF) and one ml of anisole at -15 ° C for two hours. After removal of the HF, the resin is extensively washed with ether and the peptide is extracted with glacial acetic acid (30 ml). Most of the acetic acid is removed on a rotavap and the residue is diluted in water and lyophilized. Afer purification by HPLC, the peptide is obtained.

EXAMPLE 6

Preparation of $(pGlu\text{-}Glu\text{-}Asp)_2\text{-}Pim\text{-}(Arg\text{-}CONH_2)_2$

A half gram of BOC-Tos Arg-BHA (0.5 m. M/g) is charged in the reaction vessel of a Beckman 990 synthesizer. The BOC group is removed using 40% TFA in methylene chloride. The trifluroacetic acid salt is neutralized by 10% DIEA/$CH_2Cl_2$. One mM of Bis BOC pimelic acid is coupled using 2 mM of DCC and HOBT in 15 ml of $CH_2Cl_2$ and 10 ml of DMF at room temperature. The Kaiser test is used to monitor the coupling. Any free remaining carboxyl groups are amidated using 3 mM of H-Lys-OBz. HCL; and 3 mM of DCC and HOBT in 25 ml of $CH_2Cl_2$/ DMF (15/10). After coupling the resin is extensively washed with $CH_2Cl_2$, 30% MeOH-$CH_2Cl_2$, and $CH_2Cl_2$ (25 ml x 3). The cycles of deprotection, neutralization and

coupling are repeated with the remaining amino acids in the target peptide (Asp, Glu and p-Glu). 3 mM of amino acid, DCC and HOBT are used for each coupling. Each coupling is monitored using the Kaiser test. After completion of the synthesis, the resin was dried and weighed.

The peptide resin is charged in a cleavage apparatus and cleaved using 10 ml of hydrofluoric acid (HF) and one ml of anisole at -15 °C for two hours. After removal of the HF, the resin is extensively washed with ether and the peptide is extracted with glacial acetic acid (30 ml). Most of the acetic acid is removed on a rotavap and the residue is diluted in water and lyophilized. After purification by HPLC the peptide is obtained.

EXAMPLE 7

Synthesis of Tyrosine containing analogs:

(Tyr-Glu-Asp)$_2$-Sub-(Lys)$_2$;
(pGlu-Tyr-Glu-Asp)$_2$-Sub-(Lys)$_2$;
(pGlu-Glu-Tyr-Asp)$_2$-Sub-(Lys)$_2$;
(pGlu-Glu-Asp-Tyr)$_2$-Sub-(Lys)$_2$.

Two grams of BOC-Lys(Cl-Z)-O-Resin (Peninsula Labs®, substitution 0.49 mM/g) was charged in a manual shaker vessel. After deprotection and neutralization steps, 2 mM (808 mg) of di-BOC diaminosuberic acid was coupled to the resin using 4 mM (824 mg) of dicyclohexylcarbodiimide (DCC) and 4 mM (612 mg) of 1-hydroxybenzotriazole hydrate (HOBT) in 25 ml of 50% N-methyl-2-pyrrolidinone (NMP) and dicholoromethane (DCM). The reaction was allowed to proceed overnight followed by the addition of 10 mM (4.06 g) H-Lys (Z)-OBz.HCl, 10 mM (1.29 g) diisopropylethylamine (DIEA), 10 mM (2.06 g) DCC and 10 mM (1.53 g) HOBT. After two hours, the unreacted amino groups were capped using 10% acetic anhydride in NMP/DCM (1:1). Approximately one third of the resulting BOC-Sub-Lys-resin was transferred to another reaction vessel. The major fraction of the resin is called fraction I, and minor fraction is called fraction II. The standard deprotection, neutralization and coupling cycles were used to couple BOC-Tyr (Br-Z), BOC-Asp(OBz), BOC-Glu(OBz), and p-Glu to the resin in fraction II. Five mM of amino acid, DCC and HOBt were used. Coupling was performed in 25 ml NMP/DCM (1/1) and was monitored for completion using the Kaiser test. Five mM of BOC-Asp(OBz) were coupled to the resin in fraction I. One fourth of the resulting BOC-Asp-Sub-Lys resin was transferred to another vessel (fraction III). The remaining resin is called fraction IV. Standard deprotection, neutralization and coupling cycles were used to couple BOC-Tyr (Br-Z), BOC-Glu-(OBz),and p-Glu to resin in fraction III. Five mM of amino acid, DCC and HOBt were used. Coupling was performed in 25 ml NMP/DCM (1/1) and was monitored for completion using Kaiser test. Five mM of BOC-Glu(OBz) were coupled to the resin in the major fraction (fraction IV). One third of this resin was transferred to another vessel and 5 mM of p-Glu were coupled to this fraction (fraction VI) resulting in the synthesis of pGlu-Glu-Asp-Sub-Lys-Resin. Five mM of BOC Tyr(Br-Z) were coupled to the major fraction (fraction V) and the resin was further split in halves. One half of the resin was saved as is and to the other half of the resin (fraction VII) 5 mM of p-Glu were coupled resulting in the synthesis of p-Glu-Tyr-Glu-Asp-Sub-Lys-Resin. The scheme is described on the following page:

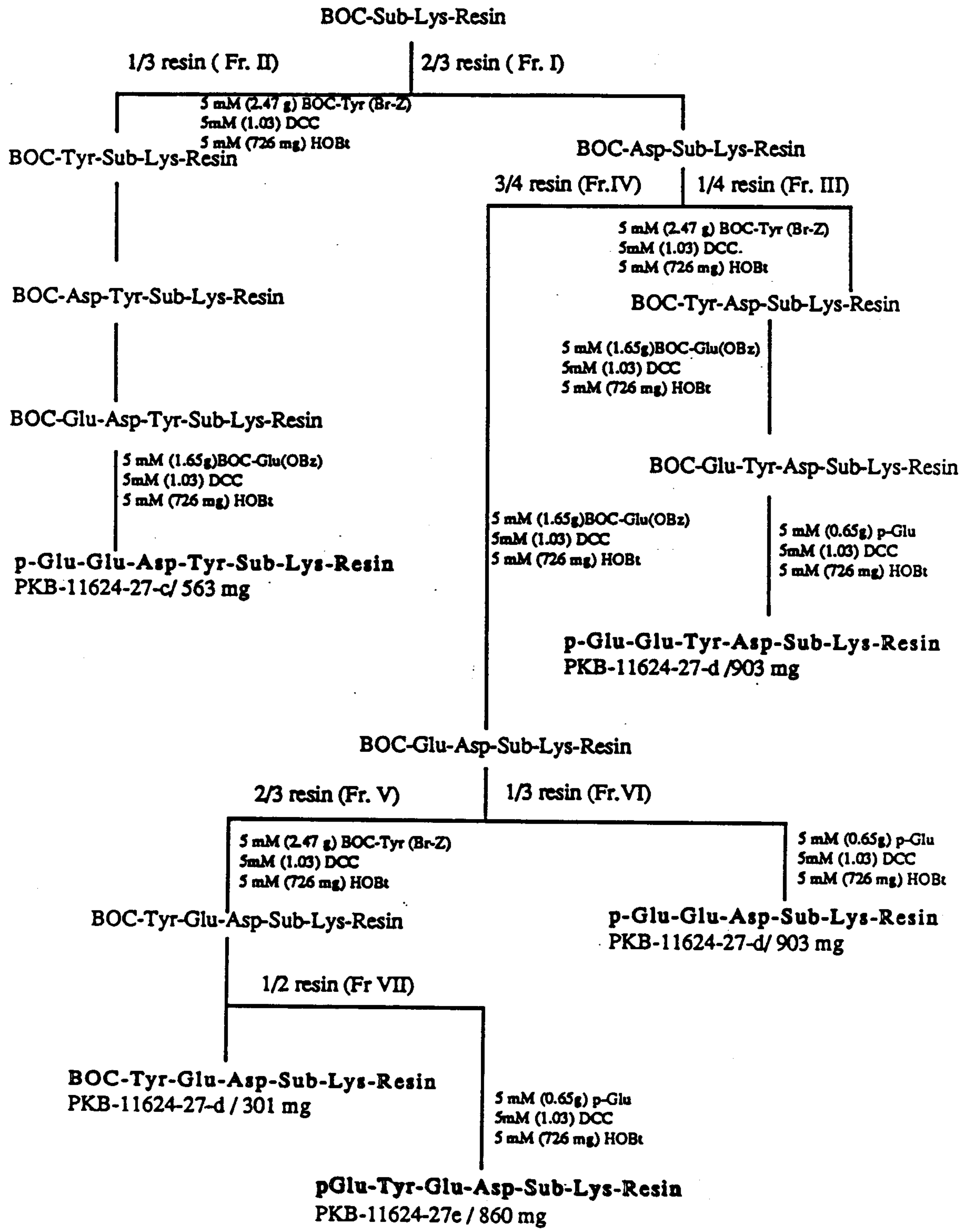


These resin peptides were deprotected and cleaved using HF/anisole at 0 °C for one hour. The crude peptides (approx. 100 mg) were purified on a C-18 Vydac 2.5 cm x 30 cm preparative column using water/0.1% trifluroacetic acid(TFA), and acetcnitrile/.01% TFA buffer system,

| | **FAB/MS** (M+H) | **Amino Acid Analysis*** | | | |
|---|---|---|---|---|---|
| | | **Asp** | **Glu** | **Sub** | **Lys** |
| (Tyr-Glu-Asp)$_2$-Sub-(Lys)$_2$ | 1274 | 2.08(2) | 2.4(2) | 1.08(1) | 2(2) |
| (pGlu-Tyr-Glu-Asp)$_2$-Sub-(Lys)$_2$ | 1497 | 2.1(2) | 3.9(4) | 1.01(1) | 2(2) |
| (pGlu-Glu-Tyr-Asp)$_2$-Sub-(Lys)$_2$ | 1497 | 2.2(2) | 3.86(4) | 0.98(1) | 2(2) |
| (pGlu-Glu-Asp-Tyr)$_2$-Sub-(Lys)$_2$ | 1497 | 2.2(2) | 4.06(4) | 1.0(2) | 2(2) |

* The number in the parenthesis indicates the theoretical ratios. The experimental ratios were determined with respect to Lys.

EXAMPLE 8

Preparation of (pGlu Glu Asp)$_2$Prc(Lys)$_2$

a) Synthesis of bis BOC-S,S'-1-3-propanediylcysteine:

Three ml of methanol were saturated with dry ammonia and 0.5 g BOC-cysteine in 0.5 ml methanol was added, followed by the addition of 0.35 ml of 1,3 dibromopropane. Ten minutes later, additional 0.5 g of BOC-cysteine in 0.5 ml methanol was added. After 4.5 hrs, the solvent was evaporated and the oily residue dissolved in water. The pH of the solution was adjusted to 9, and the solution was extracted with ether. The aqueous layer was acidified to pH 2 and extracted with ethylacetate. The organic layer was dried and evaporated to yield 1.12 g of Bis BOC-S,S-1,3-propanediylcysteine. The amino acid was used without any further purification, FAB/MS M+H = 469.

b) Preparation of (pGluGluAsp)$_2$Prc(Lys)$_2$

BOC-Lys resin (0.53 g, substitution 0.63 mM/g) was charged in a manual shaker and after deprotection and neutralization cycles, bis BOC-S,S'-1,3-propanediylcysteine (290 mg, 0.6 mM) was coupled using 1 mM (206 mg) DCC and 1 mM (153 mg) HOBt in 10 ml NMP/DCM (1/1). After two hours, the resin was washed with NMP and DCM and 2 mM (760 mg) of H-Lys (Z)-OBz was added followed by the addition of 1.5 mM (390 mg) DCC and 1.5 mM (230 mg) of HOBt in 4 ml of NMP/DCM (1/1). After 18 hrs, the resin was washed using 20 ml NMP and DCM. Normal deprotection and neutralization and coupling cycles were repeated for the coupling of BOC-Asp(OBz), BOC-Glu(OBz) and p-Glu. One mM of amino acid, DCC and HOBt were used. Coupling was done in 5 ml of NMP/DCM (1/1). Completion of the coupling was monitored using Kaiser's test. The resulting resin peptide (416 mg) was deprotectd and cleaved using 0.5 ml anisole and 8 ml of HF at 0°C for 2 hrs. HF was evaporated and the peptide resin mixture was washed with ether and extracted with glacial acetic acid. After lyophilization, 130 mg of the crude peptide was obtained. The crude peptide (61.5 mg) was purified on a C 18 Vydac preparative column using acetonitrile-water (0.1% TFA) buffer system. 16.5 Mg of pure peptide was obtained.
FAB/MS: M+H 1249.3

| Amino Acid Analysis | |
|---|---|
| Asp | 2.0(2) |
| Glu | 4.28 (4) |
| Dpc | 1.14 |
| Lys | 1.96(2) |

EXAMPLES 9 and 10

Following the procedure for bis BOC-S,S'-1,3-propanediylcysteine (Prc) bis BOC-S,S'-1,2-ethanediylcysteine (Etc) and bis BOC-S,S'-1,4-butanediylcysteine (Buc) were made.

Following the procedures given for (pGluGluAsp)$_2$PrC(Lys)$_2$, 30 mg of (pGluGluAsp)$_2$Etc(Lys)$_2$ and 17 mg of (pGluGluAsp)$_2$Buc(Lys)$_2$ were made.

EXAMPLE 11

Synthesis of (pGlu-Glu-Asp)$_2$ Sub(N-MeArg)$_2$

Hydroxy methyl resin 0.5 g (0.45 meq/g) was suspended in DCM (5 ml) and reacted with 0.5 mM (221 mg) BOC-NMe Arg, 0.5 mM (61 mg) dimethylamino pyridine, and 0.5 mM (103 mg) DCC. The acylated resin was washed with DCM (3x), NMP (3x), and DCM (4x). The unreacted hydroxy groups were capped using 0.5 ml phenylisocyanate in 20 ml of DCM. The resin was thoroughly washed with DCM and NMP. The BOC group was removed using 40% TFA/DCM. After neutralization with 10% DIEA in DCM .05 mM (20.2 mg), BOC-Sub was coupled using 0.125 mM (25.7 mg ) of DCC and 0.125 (19.1 mg) mM HOBt. After 72 hrs, the resin was washed with NMP and DCM. Normal deprotection, neutralization and coupling cycles were repeated for the coupling of BOC-Asp(OBz), BOC-Glu(OBz) and p-Glu. One mM of amino acid, DCC and HOBt were used. Coupling was done in 5 ml of NMP/DCM (1/1). Completion of the coupling was monitored using Kaiser's test. The resulting resin peptide (484 mg) was deprotected and cleaved using 0.5 ml anisole and 10 ml of HF at 0° C for 2 hrs. The HF was evaporated and the peptide resin mixture was washed with ether and extracted with glacial acetic acid. After lyophilization, 12.5 mg of the crude peptide was obtained. The crude peptide (6 mg) was purified on a C-18 Vydac preparative column using acetonitrile-water (0.1% TFA) buffer system. 2.2 mg of pure peptide was obtained.

EXAMPLE 12

Synthesis of (pGlu-Glu-Asp)$_2$ Sub(Hna)$_2$

Hydroxy methyl resin 0.5 g (0.45 meq/g) was suspended in DCM (5 ml) and reacted with 126 mg (0.335 mM) 2,N-BOC-6,N-Z-2,6 diamino-4-hexynoic acid (Hna), 40 mg (0.335) dimethylaminopyridine (DMAP), 69 mg (0.335) DCC and 50 mg (0.335) HOBt. The acylated resin was washed with DCM (3x), NMP (3x), and DCM (4x). Unreacted hydroxy groups were capped using 0.5 ml phenylisocyanate in 20 ml of DCM. The resin was thoroughly washed with DCM and NMP. The BOC group was removed using 40% TFA/DCM. After neutralization with 10% DIEA in DCM, BOC-Sub 44 mg (0.11 mM) was coupled using 50 mg (0.22 mM) of DCC and 33.6 mg (0.11 mM) HOBt. After 16 hrs, the resin was washed using NMP and DCM. Normal deprotection and neutralization and coupling cycles were repeated for the coupling of BOC Asp-(OBz), BOC-Glu(OBz) and p-Glu. One mg of amino acid, DCC and HOBt were used. Coupling was done in 5 ml of NMP/DCM (1/1). Completion of the coupling was monitored using Kaiser's test. The resulting resin peptide (608 mg) was deprotectd and cleaved using 0.6 ml anisole and 10 ml of HF at 0°C for 2 hrs. The HF was evaporated and the peptide resin mixture was washed with ether and extracted with glacial acetic acid. After lyophilization, 93.7 mg of the crude peptide was obtained. The crude peptide (20.6 mg) was purified on a C-18 Vydac preparative column using acetonitrile-water (0.1% TFA) buffer system. 7.5 Mg of pure peptide was obtained. Amino acid analysis and FAB-MS confirmed the structure.
FAB/MS: M+H 1163

| Amino Acid Analysis | |
|---|---|
| Asp | 2.0 (2) |
| Glu | 4.01 (4) |
| Sub | 2.01 (1) |
| Hna | 1.44 (2) |

EXAMPLE 13

Synthesis of (pGlu-Glu-Asp)$_2$ Adp(Lys)$_2$

a) Synthesis of Bis BOC 2,5(S,S)diamino adipic acid.

Bis-BOC 2,5 (s,s ) diaminoadipic acid was synthesized using R. Nutt's method ( J. Org. Chem. 45, 3078, 1980). To a mixture of 240 ml methanol and 80 ml pyridine, 192 mg NaOCH3 and 57.56 g BOC-Asp (OBz) were added. When the solution was homogeneous, the reaction mixture was cooled to 0°C. Using platinum electrodes, approximately 1.5 amps of current (100 volt) was passed through the reaction mixture.

The temperature of the solution was mantained between 15 ° C and 25 ° C. The disappearance of the starting material was followed by TLC (chloroform:methanol:acetic acid /95:4:1) After five hours, the reaction was stopped. The solvent is removed on a rotavap. The oily brown residue was dissolved in 150 methyl acetate. The solution was allowed to stand at room temperature for 18 hours. The precipitates were removed by filtration and the filtrate was evaporated. The crude dibenzyl ester of bis BOC 2,5 diamino suberic acid (61.7 g) was obtained. Sixty one grams of the crude product was loaded on a silica gel column packed in hexane. The column was sequentially eluted with 10%, 20%, 25% and 30% ethylacetate in hexane. The fractions were analyzed by TLC. Fractions containing the desired product were pooled and solvent was removed on a rotavap. The purified product (3.78 g) was dried in vacuo. Three grams of the dibenzyl ester were dissolved in 120 mls of methanol and hydrogenated in a Parr apparatus using 378 mg of 10% Pd/charcoal. Hydrogenation was completed in five hours. The solution was filtered and the filtrate was concentrated on a rotavap. Crude bis BOC 2,5-diamino adipic acid was purified using flash silica gel chromatography on a column packed in chloroform. The column was sequentially eluted with 98;2;.1, 98:2: .5, 95:4:1, 90;8:2, 85;10:5, and 70:20:10 Chloroform, Methanol and acetic acid. The fractions containing the desired product were pooled and concentrated on a rotavap and the residue was dried in vacuo (1.07 g) . The structure of product was confirmed using NMR, and Mass spectral data.

b) Synthesis of $(pGuGluAsp)_2Adp(Lys)_2$

A half gram of BOC-Lys (Cl-Z)-PAM resin (0.63 mM) is charged in a manual shaker vessel. The BOC group is removed using 40% TFA in methylene chloride. The trifluroacetic acid salt is neutralized by 10% DIEA/DCM. 2 MM of Bis BOC 2,5 diamino adipic acid (Adp) is coupled using 4 mM DCC and HOBt in 15 ml DCM and 15 ml NMP. Any free carboxyl groups are amidated using 3 mM H-Lys(z)-OBz and 3mg of DCC and HOBt in 30 ml DCM/NMP (1/1). After coupling, the resin is extensively washed with DCM, NMP. The cycles of deprotection, neutralization and coupling are repeated with the remaining amino acids in the target peptide (Asp,Glu and pGlu). Four mM of each aminoacid, DCC and HOBt are used for each coupling. Each coupling is monitored for completion using the Kaiser test. After completion of the synthesis, the resin peptide is charged in cleavage apparatus and cleaved using 10 ml HF and 1 ml anisole at -15 ° C for two hrs. After removal of HF the resin is extensively washed with ether and the peptide is extracted in glacial acetic acid. Most of the acetic acid is removed on a rotavap and the residue is diluted with water and lyophilized. After purification by HPLC, the desired peptide is obtained.

**Claims**

**Claims for the following Contracting States : AT, BE, CH, DE, DK, FR, GB, IT, LI, LU, NL, SE**

**1.** A compound of the following formula:

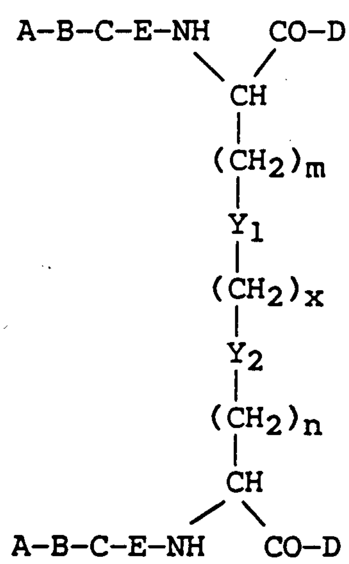

(I)

wherein:

$Y_1$ and $Y_2$ are independently $CH_2$ or S;
x is 0, 1, 2, 3 or 4;
m is 0, 1 or 2;
n is 0, 1, or 2;
A is pyroglutamic acid, proline, glutamine, tyrosine or glutamic acid;
B is glutamic acid, tyrosine or aspartic acid;
C is glutamic acids tyrosine or aspartic acid;
D is lysine, arginine, tyrosine, N-methyl arginine, diaminohexynoic acid or the carboxyamide, or hydroxy methyl derivative thereof;
E is glutamic acid, aspartic acid, tyrosine or a peptide bond;

provided that:
when $Y_1$ and $Y_2$ are S, x is 2, 3 or 4 and m and n are 1; or
when $Y_1$ and $Y_2$ are $CH_2$, x is 0, 1 or 2 and m and n are 0; or
when $Y_1$ is S and $Y_2$ is $CH_2$, x is 0 and n is 1; or
when $Y_2$ is S and $Y_1$ is $CH_2$, x is 0 and m is 1;

or a pharmaceutically acceptable salt thereof.

**2.** A compound of claim 1 wherein $Y_1$ and $Y_2$ are $CH_2$.

**3.** A compound of claim 2 wherein x is 1 or 2.

**4.** A compound of claim 1 wherein $Y_1$ is sulfur.

**5.** A compound of claim 1 wherein $Y_1$ and $Y_2$ are sulfur.

**6.** A compound of claim 3 wherein A is pyroglutamic acid, B is glutamic acid, C is aspartic acid, D is lysine and E is a peptide bond.

**7.** A compound of claim 1 which is $(pGlu\text{-}Glu\text{-}Asp)_2\text{-}Pim\text{-}(Lys)_2$.

**8.** A compound of claim 1 which is $(pGlu\text{-}Glu\text{-}Asp)_2\text{-}Sub\text{-}(Lys)_2$.

**9.** A compound of claim 1 selected from the group consisting of:
$(Tyr\text{-}Glu\text{-}Asp)_2$ $Sub(Lys)_2$
$(pGlu\text{-}Tyr\text{-}Glu\text{-}Asp)_2$ $Sub(Lys)_2$
$(pGlu\text{-}Glu\text{-}Tyr\text{-}Asp)_2$ $Sub(Lys)_2$
$(pGlu\text{-}Glu\text{-}Asp\text{-}Tyr)_2$ $Sub(Lys)_2$
$(pGlu\text{-}Glu\text{-}Asp)_2$ $Sub(N\text{-}MeArg)_2$
$(pGlu\text{-}Glu\text{-}Asp)_2$ $Sub(HNA)_2$
$(pGlu\text{-}GluAsp)_2Prc(Lys)_2$
$(pGluGluAsp)_2Etc(Lys)_2$
$(pGluGluAsp)_2Buc(Lys)_2$

**10.** A compound according to any one of claims 1 to 9 for use as a medicament.

**11.** A pharmaceutical composition which comprises a compound according to any one of claims 1 to 9 and a pharmaceutically acceptable carrier.

**12.** A process for preparing a compound of the formula I as defined in claim 1, which process comprises,

a) coupling protected amino acids to form a suitably protected compound of the formula:

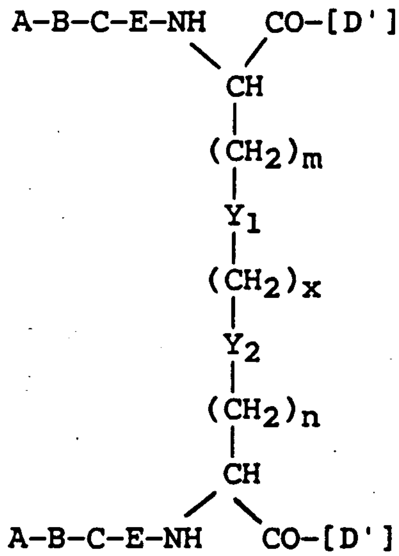

wherein:

$Y_1$ and $Y_2$ are independently $CH_2$ or S;
x is 0, 1, 2, 3 or 4;
m is 0, 1 or 2;
n is 0, 1, or 2;
A is pyroglutamic acid, proline, glutamine, tyrosine or glutamic acid;
B is glutamic acid, tyrosine or aspartic acid;
C is glutamic acid, tyrosine or aspartic acid;
E is glutamic acid, aspartic acid, tyrosine or a peptide bond;

provided that:

when $Y_1$ and $Y_2$ are S, x is 2, 3 or 4 and m and n are 1; or
when $Y_1$ and $Y_2$ are $CH_2$, x is 0, 1 or 2 and m and n are 0; or
when $Y_1$ is S and $Y_2$ is $CH_2$, x is 0 and n is 1; or
when $Y_2$ is S and $Y_1$ is $CH_2$, x is 0 and m is 1;

and [D′] is a chloromethyl, methylbenzhydylamine, benzhydrylamine or phenylacetamidomethyl resin, or D is lysine, arginine, tyrosine, N-methyl arginine, diaminohexynoic acid or the carboxyamide, or hydroxy methyl derivative thereof;

b) removing any protecting groups and, if necessary, cleaving the peptide from the resin; and
c) optionally forming a pharmaceutically acceptable salt thereof.

**13.** The use of a compound of formula I or a pharmaceutically acceptable salt thereof as defined in claim 1 in the manufacture of a medicament to stimulate the myelopoietic system.

**14.** The use of a compound of formula I or a pharmaceutically acceptable salt thereof as defined in claim 1 in the manufacture of a medicament for the treatment of viral, fungal, and bacterial infections.

**Claims for the following Contracting States: ES, GR**

**1.** A process for preparing a compound of formula I:

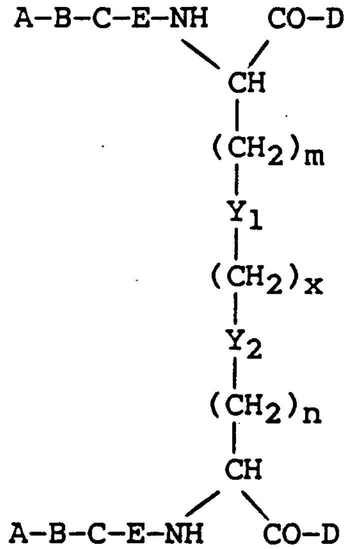

(I)

wherein:

$Y_1$ and $Y_2$ are independently $CH_2$ or S;
x is 0, 1, 2, 3 or 4;
m is 0, 1 or 2;
n is 0, 1, or 2;
A is pyroglutamic acid, proline, glutamine, tyrosine or glutamic acid;
B is glutamic acid, tyrosine or aspartic acid;
C is glutamic acid, tyrosine or aspartic acid;
D is lysine, arginine, tyrosine, N-methyl arginine, diaminohexynoic acid or the carboxyamide, or hydroxy methyl derivative thereof;
E is glutamic acid, aspartic acid, tyrosine or a peptide bond;

provided that:

when $Y_1$ and $Y_2$ are S, x is 2, 3 or 4 and m and n are 1; or
when $Y_1$ and $Y_2$ are $CH_2$, x is 0, 1 or 2 and m and n are 0; or
when $Y_1$ is S and $Y_2$ is $CH_2$, x is 0 and n is 1; or
when $Y_2$ is S and $Y_1$ is $CH_2$, x is 0 and m is 1;

or a pharmaceutically acceptable salt thereof,
which process comprises,

a) coupling protected amino acids to form a suitably protected compound of the formula:

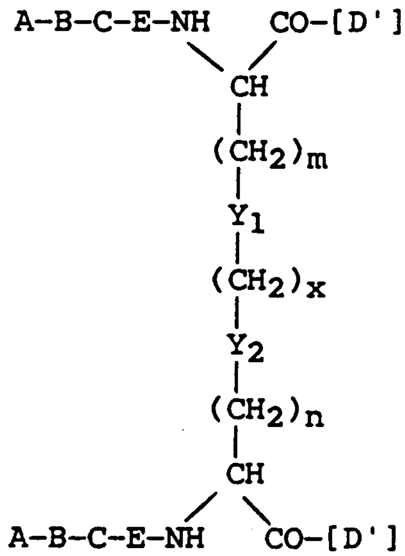

wherein A, B, C, E, $Y_1$, $Y_2$, m, x and n are as defined above for formula I and [D'] is a chloromethyl, methylbenzhydylamine, benzhydrylamine or phenylacetamidomethyl resin, or D as defined above for fomula I;

b) removing any protecting groups and, if necessary, cleaving the peptide from the resin; and

c) optionally forming a pharmaceutically acceptable salt thereof.

**2.** A process according to claim 1 for preparing a compound wherein $Y_1$ and $Y_2$ are $CH_2$.

**3.** A process according to claim 1 wherein x is 1 or 2.

**4.** A process accoridng to claim 1 wherein $Y_1$ is sulfur.

**5.** A process according to claim 1 wherein $Y_1$ and $Y_2$ are sulfur.

**6.** A process according to claim 1 wherein A is pyroglutamic acid, B is glutamic acid, C is aspartic acid, D is lysine and E is a peptide bond.

**7.** A process according to claim 1 for preparing a compound which is $(pGlu\text{-}Glu\text{-}Asp)_2$-Pim-$(Lys)_2$.

**8.** A process according to claim 1 for preparing a compound which is $(pGlu\text{-}Glu\text{-}Asp)_2$-Sub-$(Lys)_2$.

**9.** A compound according to claim 1 selected from the group consisting of:

(Tyr-Glu-Asp)$_2$ Sub(Lys)$_2$
(pGlu-Tyr-Glu-Asp)$_2$ Sub(Lys)$_2$
(pGlu-Glu-Tyr-Asp)$_2$ Sub(Lys)$_2$
(pGlu-Glu-Asp-Tyr)$_2$ Sub(Lys)$_2$
(pGlu-Glu-Asp)$_2$ Sub(N-MeArg)$_2$
(pGlu-Glu-Asp)$_2$ Sub(HNA)$_2$
(pGlu-GluAsp)$_2$Prc(Lys)$_2$
(pGluGluAsp)$_2$Etc(Lys)$_2$
(pGluGluAsp)$_2$Buc(Lys)$_2$

**10.** A process for preparing a pharmaceutical composition which comprises bringing into association a compound of the formula (I) or a pharmaceutically acceptable salt thereof as defined in claim 1 and a pharmaceutically acceptable carrier.

**11.** The use of a compound of the formula (I) or a pharmaceutically acceptable salt thereof as defined in claim 1 in the manufacture of a medicament to stimulate the myelopoietic systein.

**12.** The use of a compound of the formula (I) or a medicament for the treatment of viral, fungal and bacterial infections.

**Patentansprüche**
**Patentansprüche für folgende Vertragsstaaten : AT, BE, CH, DE, DK, FR, GB, IT, LI, LU, NL, SE**

**1.** Verbindung der folgenden Formel:

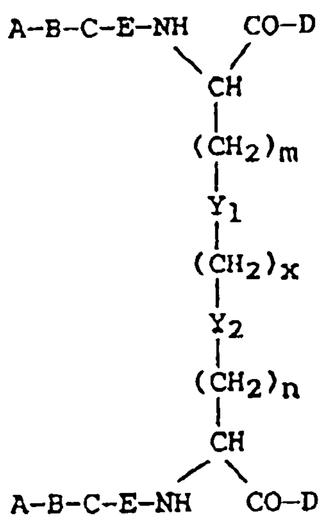

(I)

in der:
$Y_1$ und $Y_2$ unabhängig voneinander eine $CH_2$-Gruppe oder ein Schwefelatom bedeuten,
x den Wert, 0, 1, 2, 3 oder 4 hat,
m den Wert 0, 1 oder 2 hat,
n den Wert 0, 1 oder 2 hat,
A Pyroglutaminsäure, Prolin, Glutamin, Tyrosin oder Glutaminsäure bedeutet,
B Glutaminsäure, Tyrosin oder Asparaginsäure darstellt,
C Glutaminsäure, Tyrosin oder Asparaginsäure bedeutet,
D Lysin, Arginin, Tyrosin, N-Methylarginin, Diaminohexinsäure oder das Carboxyamid oder das Hydroxymethyl-derivat davon darstellt,
E Glutaminsäure, Asparaginsäure, Tyrosin oder eine Peptidbindung bedeutet,
mit der Maßgabe daß:
wenn $Y_1$ und $Y_2$ Schwefelatome sind, x den Wert 2, 3 oder 4 und m und n den Wert 1 haben, oder
wenn $Y_1$ und $Y_2$ $CH_2$-Gruppen sind, x den Wert 0, 1 oder 2 und m und n den Wert 0 haben, oder
wenn $Y_1$ ein Schwefelatom und $Y_2$ eine $CH_2$-Gruppe ist, x den Wert 0 und n den Wert 1 hat, oder
wenn $Y_2$ ein Schwefelatom und $Y_1$ eine $CH_2$-Gruppe ist, x den Wert 0 und m den Wert 1 hat,
oder ein pharmazeutisch verträgliches Salz davon.

**2.** Verbindung nach Anspruch 1, wobei $Y_1$ und $Y_2$ $CH_2$-Gruppen sind.

**3.** Verbindung nach Anspruch 2, wobei x den Wert 1 oder 2 hat.

**4.** Verbindung nach Anspruch 1, wobei $Y_1$ ein Schwefelatom ist.

**5.** Verbindung nach Anspruch 1, wobei $Y_1$ und $Y_2$ Schwefelatome sind.

**6.** Verbindung nach Anspruch 3, wobei A Pyroglutaminsäure, B Glutaminsäure, C Asparaginsäure, D Lysin und E eine Peptidbindung ist.

**7.** Verbindung nach Anspruch 1, nämlich (pGlu-Glu-Asp)$_2$-Pim-(Lys)$_2$.

**8.** Verbindung nach Anspruch 1, nämlich (pGlu-Glu-Asp)$_2$-Sub-(Lys)$_2$.

**9.** Verbindung nach Anspruch 1, ausgewählt aus:
(Tyr-Glu-Asp)$_2$Sub(Lys)$_2$
(pGlu-Tyr-Glu-Asp)$_2$Sub(Lys)$_2$
(pGlu-Glu-Tyr-Asp)$_2$Sub(Lys)$_2$
(pGlu-Glu-Asp-Tyr)$_2$Sub(Lys)$_2$
(pGlu-Glu-Asp)$_2$Sub(N-MeArg)$_2$
(pGlu-Glu-Asp)$_2$Sub(HNA)$_2$
(pGlu-Glu-Asp)$_2$Prc(Lys)$_2$
(pGlu-Glu-Asp)$_2$Etc(Lys)$_2$
(pGlu-Glu-Asp)$_2$Buc(Lys)$_2$

**10.** Verbindung nach einem der Ansprüche 1 bis 9 zur Verwendung als Medikament.

**11.** Arzneimittel, umfassend eine Verbindung nach einem der Ansprüche 1 bis 9 und einen pharmazeutisch verträglichen Träger.

**12.** Verfahren zur Herstellung einer Verbindung der Formel I gemäß der Definition in Anspruch 1, umfassend

a) Kupplung geschützter Aminosäuren zur Erzeugung einer geeignet geschützten Verbindung der Formel

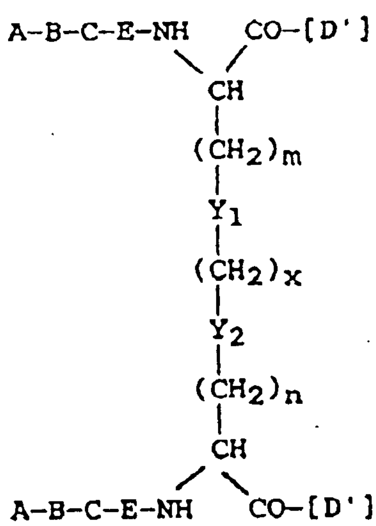

in der
$Y_1$ und $Y_2$ unabhängig voneinander eine $CH_2$-Gruppe oder ein Schwefelatom bedeuten,
x den Wert 0, 1, 2, 3 oder 4 hat,
m den Wert 0, 1 oder 2 hat,
n den Wert 0, 1 oder 2 hat,
A Pyroglutaminsäure, Prolin, Glutamin, Tyrosin oder Glutaminsäure darstellt,
B Glutaminsäure, Tyrosin oder Asparaginsäure bedeutet,
C Glutaminsäure, Tyrosin oder Asparaginsäure darstellt,
E Glutaminsäure, Asparaginsäure, Tyrosin oder eine Peptidbindung bedeutet,
mit der Maßgabe daß:
wenn $Y_1$ und $Y_2$ Schwefelatome bedeuten, x den Wert 2, 3 oder 4 und m und n den Wert 1 haben, oder
wenn $Y_1$ und $Y_2$ $CH_2$-Gruppen bedeuten, x den Wert 0, 1 oder 2 und m und n den Wert 0 haben, oder

wenn $Y_1$ ein Schwefelatom und $Y_2$ eine $CH_2$-Gruppe ist, x den Wert 0 und n den Wert 1 hat, oder wenn $Y_2$ ein Schwefelatom und $Y_1$ eine $CH_2$-Gruppe ist, x den Wert 0 und m den Wert 1 hat, und [D'] ein Chlormethyl-, Methylbenzhydrylamin-, Benzhydrylamin- oder Phenylacetamidomethylharz ist, oder D Lysin, Arginin, Tyrosin, N-Methylarginin, Diaminohexinsäure oder das Carboxyamid oder das Hxydroxymethylderivat davon bedeuten;

b) Entfernung der vorhandenen Schutzgruppen und, falls notwendig, Abspaltung des Peptids vom Harz, und

c) gegebenenfalls Erzeugung eines pharmazeutisch verträglichen Salzes davon.

**13.** Verwendung einer Verbindung der Formel I oder eines pharmazeutisch verträglichen Salzes davon gemäß der Definition in Anspruch 1 für die Herstellung eines Medikaments zur Stimulierung des Systems zur Knochenmarkbildung.

**14.** Verwendung einer Verbindung der Formel I oder eines pharmazeutisch verträglichen Salzes davon gemäß der Definition in Anspruch 1 für die Herstellung eines Medikaments zur Behandlung von durch Viren, Pilze und Bakterien verursachte Infektionen.

**Patentansprüche für folgende Vertragsstaaten : ES, GR**

**1.** Verfahren zur Herstellung einer Verbindung der folgenden Formel:

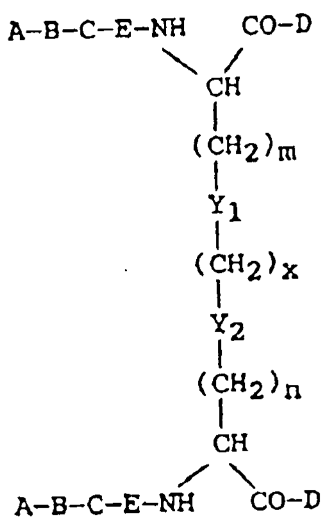

(I)

in der:
$Y_1$ und $Y_2$ unabhängig voneinander eine $CH_2$-Gruppe oder ein Schwefelatom bedeuten,
x den Wert, 0, 1, 2, 3 oder 4 hat,
m den Wert 0, 1 oder 2 hat,
n den Wert 0, 1 oder 2 hat,
A Pyroglutaminsäure, Prolin, Glutamin, Tyrosin oder Glutaminsäure bedeutet,
B Glutaminsäure, Tyrosin oder Asparaginsäure darstellt,
C Glutaminsäure, Tyrosin oder Asparaginsäure bedeutet,
D Lysin, Arginin, Tyrosin, N-Methylarginin, Diaminohexinsäure oder das Carboxyamid oder das Hydroxymethyl-derivat davon darstellt,
E Glutaminsäure, Asparaginsäure, Tyrosin oder eine Peptidbindung bedeutet,
mit der Maßgabe daß:
wenn $Y_1$ und $Y_2$ Schwefelatome sind, x den Wert 2, 3 oder 4 und m und n den Wert 1 haben, oder
wenn $Y_1$ und $Y_2$ $CH_2$-Gruppen sind, x den Wert 0, 1 oder 2 und m und n den Wert 0 haben, oder
wenn $Y_1$ ein Schwefelatom und $Y_2$ eine $CH_2$-Gruppe ist, x den Wert 0 und n den Wert 1 hat, oder
wenn $Y_2$ ein Schwefelatom und $Y_1$ eine $CH_2$-Gruppe ist, x den Wert 0 und m den Wert 1 hat,
oder eines pharmazeutisch verträglichen Salzes davon, umfassend

a) Kupplung geschützter Aminosäuren zur Erzeugung einer geeignet geschützten Verbindung der Formel

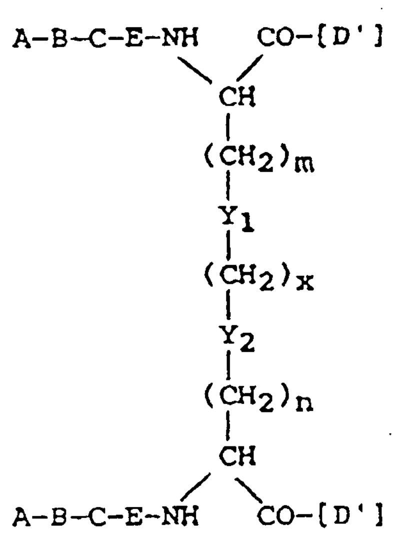

in der A, B, C, E, $Y_1$, $Y_2$, m, x und n die vorstehend für die Formel I angegebene Bedeutung haben, und [D'] ein Chlormethyl-, Methylbenzhydrylamin-, Benzhydrylamin- oder Phenylacetamidomethylharz ist, oder D die vorstehend für Formel I angegebene Bedeutung hat,

b) Entfernung der vorhandenen Schutzgruppen und, falls notwendig, Abspaltung des Peptids vom Harz, und

c) gegebenenfalls Erzeugung eines pharmazeutisch verträglichen Salzes davon.

**2.** Verfahren nach Anspruch 1 zur Herstellung einer Verbindung, in der $Y_1$ und $Y_2$ $CH_2$-Gruppen sind.

**3.** Verfahren nach Anspruch 1 zur Herstellung einer Verbindung, in der x den Wert 1 oder 2 hat.

**4.** Verfahren nach Anspruch 1 zur Herstellung einer Verbindung, in der $Y_1$ ein Schwefelatom ist.

**5.** Verfahren nach Anspruch 1, wobei $Y_1$ und $Y_2$ Schwefelatome sind.

**6.** Verfahren nach Anspruch 1, wobei A Pyroglutaminsäure, B Glutaminsäure, C Asparaginsäure, D Lysin und E eine Peptidbindung ist.

**7.** Verfahren nach Anspruch 1 zur Herstellung der Verbindung $(pGlu\text{-}Glu\text{-}Asp)_2\text{-}Pim\text{-}(Lys)_2$.

**8.** Verfahren nach Anspruch 1 zur Herstellung der Verbindung $(pGlu\text{-}Glu\text{-}Asp)_2\text{-}Sub\text{-}(Lys)_2$.

**9.** Verbindung nach Anspruch 1, ausgewählt aus:

$(Tyr\text{-}Glu\text{-}Asp)_2Sub(Lys)_2$
$(pGlu\text{-}Tyr\text{-}Glu\text{-}Asp)_2Sub(Lys)_2$
$(pGlu\text{-}Glu\text{-}Tyr\text{-}Asp)_2Sub(Lys)_2$
$(pGlu\text{-}Glu\text{-}Asp\text{-}Tyr)_2Sub(Lys)_2$
$(pGlu\text{-}Glu\text{-}Asp)_2Sub(N\text{-}MeArg)_2$
$(pGlu\text{-}Glu\text{-}Asp)_2Sub(HNA)_2$
$(pGlu\text{-}Glu\text{-}Asp)_2Prc(Lys)_2$
$(pGlu\text{-}Glu\text{-}Asp)_2Etc(Lys)_2$
$(pGlu\text{-}Glu\text{-}Asp)_2Buc(Lys)_2$

**10.** Verfahren zur Herstellung eines Arzneimittels, umfassend das Zusammenbringen einer Verbindung der Formel (I) oder eines pharmazeutischen Salzes davon gemäß der Definition in Anspruch 1 und eines pharmazeutisch verträglichen Trägers.

**11.** Verwendung einer Verbindung der Formel (I) oder eines pharmazeutisch verträglichen Salzes davon gemäß der Definition in Anspruch 1 für die Herstellung eines Medikaments zur Stimulierung des Systems zur Knochenmarkbildung.

**12.** Verwendung einer Verbindung der Formel (I) oder eines Medikaments zur Behandlung von durch Viren, Pilze und Bakterien verursachte Infektionen.

**Revendications**

**Revendications pour les Etats contractants suivants : AT, BE, CH, DE, DK, FR, GB, IT, LI, LU, NL, SE**

**1.** Composé représenté par la formule suivante :

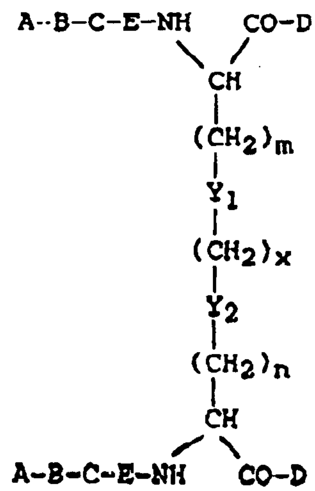

(I)

dans laquelle :

| | |
|---|---|
| $Y_1$ et $Y_2$ | sont indépendamment un groupe $CH_2$ ou S; |
| x | est 0, 1, 2, 3 ou 4; |
| m | est 0, 1 ou 2; |
| n | est 0, 1 ou 2; |
| A | est l'acide pyroglutamique, la proline, la glutamine, la tyrosine ou l'acide glutamique; |
| B | est l'acide glutamique, la tyrosine ou l'acide aspartique; |
| C | est l'acide glutamique, la tyrosine ou l'acide aspartique; |
| D | est la lysine, l'arginine, la tyrosine, la N-méthyl arginine, l'acide diaminohexynoïque ou son dérivé carboxyamide ou hydroxy méthyle; |
| E | est l'acide glutamique, l'acide aspartique, la tyrosine ou une liaison peptide; |

à condition que :

lorsque $Y_1$ et $Y_2$ sont S, x soit 2, 3 ou 4 et m et n soient 1; ou

lorsque $Y_1$ et $Y_2$ sont un groupe $CH_2$, x soit 0, 1 ou 2 et m et n soient 0; ou

lorsque $Y_1$ est S et $Y_2$ est un groupe $CH_2$, x soit 0 et n soit 1; ou

lorsque $Y_2$ est S et $Y_1$ est un groupe $CH_2$, x soit 0 et m soit 1; ou sel de celui-ci acceptable du point de vue pharmaceutique.

**2.** Composé suivant la revendication 1, dans lequel $Y_1$ et $Y_2$ sont un groupe $CH_2$.

**3.** Composé suivant la revendication 2, dans lequel x est 1 ou 2.

**4.** Composé suivant la revendication 1, dans lequel $Y_1$ est un atome de soufre.

**5.** Composé suivant la revendication 1, dans lequel $Y_1$ et $Y_2$ sont chacun un atome de soufre.

**6.** Composé suivant la revendication 3, dans lequel A est l'acide pyroglutamique, B est l'acide glutamique, C est l'acide aspartique, D est la lysine et E est une liaison peptide.

**7.** Composé suivant la revendication 1, qui est $(pGlu\text{-}Glu\text{-}Asp)_2\text{-}Pim\text{-}(Lys)_2$.

**8.** Composé suivant la revendication 1, qui est $(pGlu\text{-}Glu\text{-}Asp)_2\text{-}Sub\text{-}(Lys)_2$.

**9.** Composé suivant la revendication 1, sélectionné dans le groupe consistant en :
$(Tyr\text{-}Glu\text{-}Asp)_2\text{-}Sub\text{-}(Lys)_2$,
$(pGlu\text{-}Tyr\text{-}Glu\text{-}Asp)_2\text{-}Sub\text{-}(Lys)_2$,
$(pGlu\text{-}Glu\text{-}Tyr\text{-}Asp)_2\text{-}Sub\text{-}(Lys)_2$,
$(pGlu\text{-}Glu\text{-}Asp\text{-}Tyr)_2\text{-}Sub\text{-}(Lys)_2$,
$(pGlu\text{-}Glu\text{-}Asp)_2\text{-}Sub\text{-}(N\text{-}MeArg)_2$,
$(pGlu\text{-}Glu\text{-}Asp)_2\text{-}Sub\text{-}(HNA)_2$,
$(pGlu\text{-}Glu\text{-}Asp)_2\text{-}Prc\text{-}(Lys)_2$,
$(pGlu\text{-}Glu\text{-}Asp)_2\text{-}Etc\text{-}(Lys)_2$,
$(pGlu\text{-}Glu\text{-}Asp)_2\text{-}Buc\text{-}(Lys)_2$.

**10.** Composé suivant l'une quelconque des revendications 1 à 9 utile comme médicament.

**11.** Composition pharmaceutique qui comprend un composé suivant l'une quelconque des revendications 1 à 9 et un support acceptable du point de vue pharmaceutique.

**12.** Procédé pour préparer un composé de formula (I) telle que définie dans la revendication 1, qui comprend :

(a) le couplage d'acides aminés protégés pour former un composé protégé représenté par la formule :

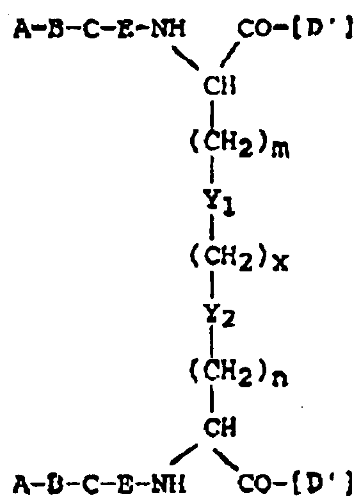

dans laquelle :

| | |
|---|---|
| $Y_1$ et $Y_2$ | sont indépendamment un groupe $CH_2$ ou S; |
| x | est 0, 1, 2, 3 ou 4; |
| m | est 0, 1 ou 2; |
| n | est 0, 1 ou 2; |
| A | est l'acide pyroglutamique, la proline, la glutamine, la tyrosine ou l'acide glutamique; |
| B | est l'acide glutamique, la tyrosine ou l'acide aspartique; |
| C | est l'acide glutamique, la tyrosine ou l'acide aspartique; |
| E | est l'acide glutamique, l'acide aspartique, la tyrosine ou une liaison peptide; |

à condition que :
lorsque $Y_1$ et $Y_2$ sont S, x soit 2, 3 ou 4 et m et n soient 1; ou
lorsque $Y_1$ et $Y_2$ sont un groupe $CH_2$, x soit 0, 1 ou 2 et m et n soient 0; ou
lorsque $Y_1$ est S et $Y_2$ est un groupe $CH_2$, x soit 0 et n soit 1: ou
lorsque $Y_2$ est S et $Y_1$ est un groupe $CH_2$, x soit 0 et m soit 1: et

[D'] est une résine chlorométhyle, méthylbenzhydrylamine, benzhydrylamine ou phénylacétamidométhyle, ou D qui est la lysine, l'arginine, la tyrosine, la N-méthyl arginine, l'acide

diaminohexynoïque ou son dérivé carboxyamide ou hydroxy méthyle;

(b) l'élimination de quelconques groupes protecteurs et, si cela est nécessaire, la séparation du peptide de la résine par coupure; et

(c) la formation éventuelle d'un sel de celui-ci acceptable du point de vue pharmaceutique.

**13.** Utilisation d'un composé de formule (I) ou d'un sel de celui-ci acceptable du point de vue pharmaceutique tel que défini dans la revendication 1, dans la fabrication d'un médicament pour stimuler le système myélopoïétique.

**14.** Utilisation d'un composé de formule (I) ou d'un sel de celui-ci acceptable du point de vue pharmaceutique tel que défini dans la revendication 1, dans la fabrication d'un médicament pour le traitement d'infections virales, fongiques et bactériennes.

**Revendications pour les Etats contractants suivants : ES, GR**

**1.** Procédé pour préparer un composé représenté par la formule :

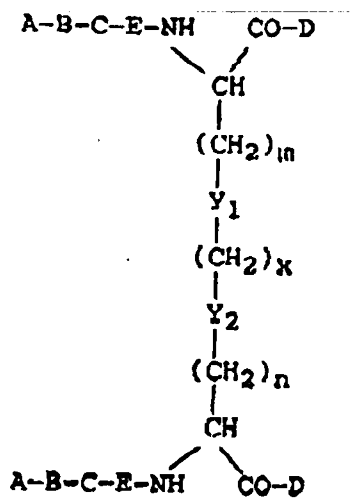

(I)

dans laquelle :

| | |
|---|---|
| $Y_1$ et $Y_2$ | sont indépendamment un groupe $CH_2$ ou S; |
| x | est 0, 1, 2, 3 ou 4; |
| m | est 0, 1 ou 2; |
| n | est 0, 1 ou 2; |
| A | est l'acide pyroglutamique, la proline, la glutamine, la tyrosine ou l'acide glutamique; |
| B | est l'acide glutamique, la tyrosine ou l'acide aspartique; |
| C | est l'acide glutamique, la tyrosine ou l'acide aspartique; |
| D | est la lysine, l'arginine, la tyrosine, la N-méthyl arginine, l'acide diaminohexynoïque ou son dérivé carboxyamide ou hydroxy méthyle; |
| E | est l'acide glutamique, l'acide aspartique, la tyrosine ou une liaison peptide; |

à condition que :

lorsque $Y_1$ et $Y_2$ sont S, x soit 2, 3 ou 4, et m et n soient 1; ou

lorsque $Y_1$ et $Y_2$ sont un groupe $CH_2$, x soit 0, 1 ou 2 et m et n soient 0; ou

lorsque $Y_1$ est S et $Y_2$ est un groupe $CH_2$, x soit 0 et n soit 1; ou

lorsque $Y_2$ est S et $Y_1$ est un groupe $CH_2$, x soit 0 et m soit 1; ou un sel de celui-ci acceptable du point de vue pharmaceutique,

lequel procédé comprend:

(a) le couplage d'acides aminés protégés pour former un composé protégé représenté par la formule :

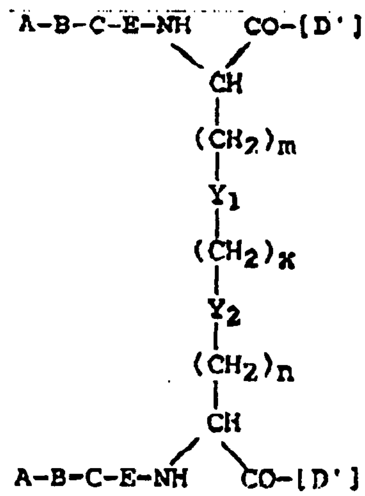

dans laquelle A, B, C, E, $Y_1$, $Y_2$, m, x et n sont tels que définis pour la formule (T), et [D'] est une résine chlorométhyle, méthylbenzhydrylamine, benzhydzylamine ou phénylacétamidométhyle, ou D tel que defini plus haut pour la formule (I);

(b) l'élimination de quelconques groupes protecteurs et, si cela est nécessaire, la séparation du peptide de la résine par coupure; et

(c) la formation éventuelle d'un sel de celui-ci acceptable du point de vue pharmaceutique.

**2.** Procédé suivant la revendication 1, pour préparer un composé dans lequel $Y_1$ et $Y_2$ sont un groupe $CH_2$.

**3.** Procédé suivant la revendication 1, pour préparer un composé dans lequel x est 1 ou 2.

**4.** Procédé suivant la revendication 1, pour préparer un composé dans lequel $Y_1$ est un atome de soufre.

**5.** Procédé suivant la revendication 1, pour préparer un composé dans lequel $Y_1$ et $Y_2$ sont chacun un atome de soufre.

**6.** Procédé suivant la revendication 1, pour préparer un composé dans lequel A est l'acide pyroglutamique, B est l'acide glutamique, C est l'acide aspartique, D est la lysine et E est une liaison peptide.

**7.** Procédé suivant la revendication 1, pour préparer un composé qui est $(pGlu\text{-}Glu\text{-}Asp)_2\text{-}Pim\text{-}(Lys)_2$.

**8.** Procédé suivant la revendication 1, pour préparer un composé qui est $(pGlu\text{-}Glu\text{-}Asp)_2\text{-}Sub\text{-}(Lys)_2$.

**9.** Procédé suivant la revendication 1, pour préparer un composé sélectionné dans le groupe consistant en :

$(Tyr\text{-}Glu\text{-}Asp)_2\text{-}Sub\text{-}(Lys)_2$,
$(pGlu\text{-}Tyr\text{-}Glu\text{-}Asp)_2\text{-}Sub\text{-}(Lys)_2$,
$(pGlu\text{-}Glu\text{-}Tyr\text{-}Asp)_2\text{-}Sub\text{-}(Lys)_2$,
$(pGlu\text{-}Glu\text{-}Asp\text{-}Tyr)_2\text{-}Sub\text{-}(Lys)_2$,
$(pGlu\text{-}Glu\text{-}Asp)_2\text{-}Sub\text{-}(N\text{-}MeArg)_2$,
$(pGlu\text{-}Glu\text{-}Asp)_2\text{-}Sub\text{-}(HNA)_2$,
$(pGlu\text{-}Glu\text{-}Asp)_2\text{-}Prc\text{-}(Lys)_2$,
$(pGlu\text{-}Glu\text{-}Asp)_2\text{-}Etc\text{-}(Lys)_2$,
$(pGlu\text{-}Glu\text{-}Asp)_2\text{-}Buc\text{-}(Lys)_2$.

**10.** Procédé pour préparer une composition pharmaceutique qui comprend la mise on association d'un composé de formule (I) ou d'un sel de celui-ci acceptable du point de vue pharmaceutique tel que défini dans la revendication 1, et d'un support acceptable du point de vue pharmaceutique.

**11.** Utilisation d'un composé de formule (I) ou d'un sel de celui-ci acceptable du point de vue pharmaceutique tel que défini dans la revendication 1, dans la fabrication d'un médicament pour stimuler le système myélopoïétique.

**12.** Utilisation d'un composé de formule (I) ou d'un médicament pour le traitement d'infections virales, fongiques et bactériennes.